# EUROPEAN PATENT SPECIFICATION

(11) **EP 2 496 249 B1**
(45) Date of publication and mention of the grant of the patent: **09.03.2016**
(21) Application number: 10828932.3
(22) Date of filing: 28.10.2010
(51) Int. Cl.: A61K 38/26, A61P 11/00, A61P 25/00

(54) **GLP-1 RECEPTOR AGONIST FOR USE IN TREATING OBSTRUCTIVE SLEEP APNEA**
GLP-1-REZEPTOR-AGONISTEN ZUR VERWENDUNG IN DER BEHANDLUNG VON OBSTRUKTIVER SCHLAFAPNOE
AGONISTE DU RÉCEPTEUR DU GLP-1 POUR L'UTILISATION DANS LE TRAITEMENT DE L'APNÉE OBSTRUCTIVE DU SOMMEIL

(30) Priority: 03.11.2009 US 257603 P
(43) Date of publication of application: 12.09.2012
(73) Proprietor: Amylin Pharmaceuticals, LLC, Wilmington, DE 19850 (US); Eli Lilly and Company, Indianapolis, IN 46285 (US)
(72) Inventor: VAN CAUTER, Eve, San Diego, CA 92121 (US)
(74) Representative: Raynor, Stuart Andrew
(86) International application number: PCT/US2010/054553
(87) International publication number: WO 2011/056713

(56) References cited:
- US-A- 5 846 937
- US-A1- 2003 087 821
- US-A1- 2008 146 490
- US-A1- 2008 200 376
- A. N. VGONTZAS ET AL: "Metabolic disturbances in obesity versus sleep apnoea: the importance of visceral obesity and insulin resistance", JOURNAL OF INTERNAL MEDICINE, vol. 2541-13, no. 1, 1 July 2003 (2003-07-01), pages 32-44, XP55071323, ISSN: 0954-6820, DOI: 10.1046/j.1365-2796.2003.01177.x
- IDRIS ET AL.: 'Obstructive Sleep Apnoea in Patients with Type 2 Diabetes: Aetiology and Implications for Clinical Care.' DIABETES, OBESITY AND METABOLISM. vol. 11, no. 8, 07 June 2009, pages 733 - 741, XP008155762

### SEQUENCE LISTING

<110> AMYLIN PHARMACEUTICALS, INC. ELI LILLY AND COMPANY
<120> GLP-1 RECEPTOR AGONIST COMPOUNDS FOR OBSTRUCTIVE SLEEP APNEA
<130> N.116849
<140> EP 10828932.3
   <141> 2010-10-28
<150> US 61/257,603
   <151> 2009-11-03
<160> 45
<170> PatentIn version 3.5
<210> 1
   <211> 39
   <212> PRT
   <213> Heloderma suspectum
<220>
   <221> MOD_RES
   <222> (39)..(39)
   <223> AMIDATION (C-term amidated)
<400> 1
   His Gly Glu Gly Thr Phe Thr Ser Asp Leu Ser Lys Gln Met Glu Glu
   1               5                   10                  15
   Glu Ala Val Arg Leu Phe Ile Glu Trp Leu Lys Asn Gly Gly Pro Ser
               20                  25                  30
   Ser Gly Ala Pro Pro Pro Ser
           35
<210> 2
   <211> 39
   <212> PRT
   <213> Heloderma horridum
<220>
   <221> MOD_RES
   <222> (39)..(39)
   <223> AMIDATION (C-term is amidated)
<400> 2
   His Ser Asp Gly Thr Phe Thr Ser Asp Leu Ser Lys Gln Met Glu Glu
   1 5 10 15
   Glu Ala Val Arg Leu Phe Ile Glu Trp Leu Lys Asn Gly Gly Pro Ser
   20 25 30
   Ser Gly Ala Pro Pro Pro Ser
   35
<210> 3
   <211> 39
   <212> PRT
   <213> Artificial Sequence
<220>
   <223> Description of Artificial Sequence: Synthetic polypeptide
<220>
   <221> MOD_RES
   <222> (39)..(39)
   <223> AMIDATION (C-term may or may not be amidated)
<400> 3
   His Gly Glu Gly Thr Phe Thr Ser Asp Leu Ser Lys Gln Leu Glu Glu
   1 5 10 15
   Glu Ala Val Arg Leu Phe Ile Glu Trp Leu Lys Asn Gly Gly Pro Ser
   20 25 30
   Ser Gly Ala Pro Pro Pro Ser
   35
<210> 4
   <211> 39
   <212> PRT
   <213> Artificial Sequence
<220>
   <223> Description of Artificial Sequence: Synthetic polypeptide
<220>
   <221> MOD_RES
   <222> (39)..(39)
   <223> AMIDATION (C-term may or may not be amidated)
<400> 4
   His Gly Glu Gly Thr Phe Thr Ser Asp Leu Ser Lys Gln Leu Glu Glu
   1 5 10 15
   Glu Ala Val Arg Leu Phe Ile Glu Phe Leu Lys Asn Gly Gly Pro Ser
   20 25 30
   Ser Gly Ala Pro Pro Pro Ser
   35
<210> 5
   <211> 39
   <212> PRT
   <213> Artificial Sequence
<220>
   <223> Description of Artificial Sequence: Synthetic polypeptide
<220>
   <221> MOD_RES
   <222> (39)..(39)
   <223> AMIDATION (C-term may or may not be amidated)
<400> 5
   His Gly Glu Gly Thr Phe Thr Ser Asp Leu Ser Lys Gln Leu Glu Glu
   1 5 10 15
   Glu Ala Ala Arg Leu Phe Ile Glu Phe Leu Lys Asn Gly Gly Pro Ser
   20 25 30
   Ser Gly Ala Pro Pro Pro Ser
   35
<210> 6
   <211> 30
   <212> PRT
   <213> Artificial Sequence
<220>
   <223> Description of Artificial Sequence: Synthetic polypeptide
<220>
   <221> MOD_RES
   <222> (30)..(30)
   <223> AMIDATION (C-term may or may not be amidated)
<400> 6
   His Gly Glu Gly Thr Phe Thr Ser Asp Leu Ser Lys Gln Met Glu Glu
   1 5 10 15
   Glu Ala Val Arg Leu Phe Ile Glu Trp Leu Lys Asn Gly Gly
   20 25 30
<210> 7
   <211> 30
   <212> PRT
   <213> Artificial Sequence
<220>
   <223> Description of Artificial Sequence: Synthetic polypeptide
<220>
   <221> MOD_RES
   <222> (30).. (30)
   <223> AMIDATION (C-term may or may not be amidated)
<400> 7
   His Gly Glu Gly Thr Phe Thr Ser Asp Leu Ser Lys Gln Leu Glu Glu
   1               5                   10                  15
   Glu Ala Val Arg Leu Phe Ile Glu Trp Leu Lys Asn Gly Gly
               20                  25                  30
<210> 8
   <211> 30
   <212> PRT
   <213> Artificial Sequence
<220>
   <223> Description of Artificial Sequence: Synthetic polypeptide
<220>
   <221> MOD_RES
   <222> (30)..(30)
   <223> AMIDATION (C-term may or may not be amidated)
<400> 8
   His Gly Glu Gly Thr Phe Thr Ser Asp Leu Ser Lys Gln Leu Glu Glu
   1               5                   10                  15
   Glu Ala Val Arg Leu Phe Ile Glu Phe Leu Lys Asn Gly Gly
               20                  25                  30
<210> 9
   <211> 30
   <212> PRT
   <213> Artificial Sequence
<220>
   <223> Description of Artificial Sequence: Synthetic polypeptide
<220>
   <221> MOD_RES
   <222> (30)..(30)
   <223> AMIDATION (C-term may or may not be amidated)
<400> 9
   His Gly Glu Gly Thr Phe Thr Ser Asp Leu Ser Lys Gln Leu Glu Glu
   1 5 10 15
   Glu Ala Ala Arg Leu Phe Ile Glu Phe Leu Lys Asn Gly Gly
   20 25 30
<210> 10
   <211> 28
   <212> PRT
   <213> Artificial Sequence
<220>
   <223> Description of Artificial Sequence: Synthetic peptide
<220>
   <221> MOD_RES
   <222> (28)..(28)
   <223> AMIDATION (C-term may or may not be amidated)
<400> 10
   His Gly Glu Gly Thr Phe Thr Ser Asp Leu Ser Lys Gln Met Glu Glu
   1 5 10 15
   Glu Ala Val Arg Leu Phe Ile Glu Trp Leu Lys Asn
   20 25
<210> 11
   <211> 28
   <212> PRT
   <213> Artificial Sequence
<220>
   <223> Description of Artificial Sequence: Synthetic peptide
<220>
   <221> MOD_RES
   <222> (28)..(28)
   <223> AMIDATION (C-term may or may not be amidated)
<400> 11
   His Gly Glu Gly Thr Phe Thr Ser Asp Leu Ser Lys Gln Leu Glu Glu
   1 5 10 15
   Glu Ala Val Arg Leu Phe Ile Glu Trp Leu Lys Asn
   20 25
<210> 12
   <211> 28
   <212> PRT
   <213> Artificial Sequence
<220>
   <223> Description of Artificial Sequence: Synthetic peptide
<220>
   <221> MOD_RES
   <222> (28)..(28)
   <223> AMIDATION (C-term may or may not be amidated)
<400> 12
   His Gly Glu Gly Thr Phe Thr Ser Asp Leu Ser Lys Gln Leu Glu Glu
   1               5                   10                  15
   Glu Ala Val Arg Leu Phe Ile Glu Phe Leu Lys Asn
               20                  25
<210> 13
   <211> 28
   <212> PRT
   <213> Artificial Sequence
<220>
   <223> Description of Artificial Sequence: Synthetic peptide
<220>
   <221> MOD_RES
   <222> (28)..(28)
   <223> AMIDATION (C-term may or may not be amidated)
<400> 13
   His Gly Glu Gly Thr Phe Thr Ser Asp Leu Ser Lys Gln Leu Glu Glu
   1               5                   10                  15
   Glu Ala Ala Arg Leu Phe Ile Glu Phe Leu Lys Asn
               20                  25
<210> 14
   <211> 39
   <212> PRT
   <213> Artificial Sequence
<220>
   <223> Description of Artificial Sequence: Synthetic polypeptide
<220>
   <221> MOD_RES
   <222> (39)..(39)
   <223> AMIDATION (C-term may or may not be amidated)
<400> 14
   His Gly Glu Gly Thr Phe Thr Ser Asp Leu Ser Lys Gln Leu Glu Glu
   1               5                   10                  15
   Lys Ala Ala Lys Glu Phe Ile Glu Phe Leu Lys Gln Gly Gly Pro Ser
               20                  25                  30
   Ser Gly Ala Pro Pro Pro Ser
           35
<210> 15
   <211> 39
   <212> PRT
   <213> Artificial Sequence
<220>
   <223> Description of Artificial Sequence: Synthetic polypeptide
<220>
   <221> MOD_RES
   <222> (39)..(39)
   <223> AMIDATION (C-term may or may not be amidated)
<400> 15
   His Gly Glu Gly Thr Phe Thr Ser Asp Leu Ser Lys Gln Leu Glu Glu
   1               5                   10                  15
   Lys Ala Ala Lys Glu Phe Ile Glu Trp Leu Lys Gln Gly Gly Pro Ser
               20                  25                  30
   Ser Gly Ala Pro Pro Pro Ser
           35
<210> 16
   <211> 39
   <212> PRT
   <213> Artificial Sequence
<220>
   <223> Description of Artificial Sequence: Synthetic polypeptide
<220>
   <221> MOD_RES
   <222> (14)..(14)
   <223> Octyl-Gly
<220>
   <221> MOD_RES
   <222> (39)..(39)
   <223> AMIDATION (C-term may or may not be amidated)
<400> 16
   His Gly Glu Gly Thr Phe Thr Ser Asp Leu Ser Lys Gln Gly Glu Glu
   1 5 10 15
   Glu Ala Val Arg Leu Phe Ile Glu Trp Leu Lys Gln Gly Gly Pro Ser
   20 25 30
   Ser Gly Ala Pro Pro Pro Ser
   35
<210> 17
   <211> 39
   <212> PRT
   <213> Artificial Sequence
<220>
   <223> Description of Artificial Sequence: Synthetic polypeptide
<220>
   <221> MOD_RES
   <222> (34)..(34)
   <223> Octyl-Gly
<220>
   <221> MOD_RES
   <222> (39)..(39)
   <223> AMIDATION (C-term may or may not be amidated)
<400> 17
   His Gly Glu Gly Thr Phe Thr Ser Asp Leu Ser Lys Gln Leu Glu Glu
   1 5 10 15
   Glu Ala Val Arg Leu Phe Ile Glu Trp Leu Lys Gln Gly Gly Pro Ser
   20 25 30
   Ser Gly Ala Pro Pro Pro Ser
   35
<210> 18
   <211> 37
   <212> PRT
   <213> Artificial Sequence
<220>
   <223> Description of Artificial Sequence: Synthetic polypeptide
<220>
   <221> MOD_RES
   <222> (37)..(37)
   <223> AMIDATION (C-term may or may not be amidated)
<400> 18
   His Gly Glu Phe Thr Phe Thr Ser Asp Leu Ser Lys Gln Leu Glu Glu
   1 5 10 15
   Glu Ala Val Arg Leu Phe Ile Glu Trp Leu Lys Gln Gly Gly Pro Ser
   20 25 30
   Lys Glu Ile Ile Ser
   35
<210> 19
   <211> 39
   <212> PRT
   <213> Artificial Sequence
<220>
   <223> Description of Artificial Sequence: Synthetic polypeptide
<220>
   <221> MOD_RES
   <222> (39)..(39)
   <223> AMIDATION (C-term may or may not be amidated)
<400> 19
   His Gly Glu Phe Thr Phe Thr Ser Asp Leu Ser Lys Gln Leu Glu Glu
   1 5 10 15
   Lys Ala Ala Lys Glu Phe Ile Glu Trp Leu Lys Gln Gly Gly Pro Ser
   20 25 30
   Ser Gly Ala Pro Pro Pro Ser
   35
<210> 20
   <211> 39
   <212> PRT
   <213> Artificial Sequence
<220>
   <223> Description of Artificial Sequence: Synthetic polypeptide
<220>
   <221> MOD_RES
   <222> (39)..(39)
   <223> AMIDATION (C-term may or may not be amidated)
<400> 20
   His Gly Glu Gly Thr Phe Thr Ser Asp Leu Val Lys Ile Leu Glu Ala
   1               5                   10                  15
   Glu Ala Val Arg Lys Phe Ile Glu Phe Leu Lys Asn Gly Gly Pro Ser
               20                  25                  30
   Ser Gly Ala Pro Pro Pro Ser
           35
<210> 21
   <211> 40
   <212> PRT
   <213> Artificial Sequence
<220>
   <223> Description of Artificial Sequence: Synthetic polypeptide
<220>
   <221> MOD_RES
   <222> (40)..(40)
   <223> AMIDATION (C-term may or may not be amidated)
<400> 21
   His Gly Glu Gly Thr Phe Thr Ser Asp Leu Ser Lys Gln Met Glu Glu
   1               5                   10                  15
   Glu Ala Val Arg Leu Phe Ile Glu Trp Leu Lys Asn Gly Gly Pro Ser
               20                  25                  30
   Ser Gly Ala Pro Pro Pro Ser Lys
           35                  40
<210> 22
   <211> 31
   <212> PRT
   <213> Homo sapiens
<220>
   <221> MOD_RES
   <222> (31)..(31)
   <223> AMIDATION (C-term may or may not be amidated)
<400> 22
   His Ala Glu Gly Thr Phe Thr Ser Asp Val Ser Ser Tyr Leu Glu Gly
   1               5                   10                  15
   Gln Ala Ala Lys Glu Phe Ile Ala Trp Leu Val Lys Gly Arg Gly
               20                  25                  30
<210> 23
   <211> 30
   <212> PRT
   <213> Artificial Sequence
<220>
   <223> Description of Artificial Sequence: Synthetic polypeptide
<220>
   <221> MOD_RES
   <222> (30)..(30)
   <223> AMIDATION (C-term may or may not be amidated)
<400> 23
   His Ala Glu Gly Thr Phe Thr Ser Asp Val Ser Ser Tyr Leu Glu Gly
   1               5                   10                  15
   Gln Ala Ala Lys Glu Phe Ile Ala Trp Leu Val Lys Gly Arg
               20                  25                  30
<210> 24
   <211> 30
   <212> PRT
   <213> Artificial Sequence
<220>
   <223> Description of Artificial Sequence: Synthetic polypeptide
<220>
   <221> MOD_RES
   <222> (2)..(2)
   <223> Aib
<220>
   <221> MOD_RES
   <222> (29)..(29)
   <223> Aib
<220>
   <221> MOD_RES
   <222> (30)..(30)
   <223> AMIDATION (C-term may or may not be amidated)
<400> 24
   His Xaa Glu Gly Thr Phe Thr Ser Asp Val Ser Ser Tyr Leu Glu Gly
   1               5                   10                  15
   Gln Ala Ala Arg Glu Phe Ile Ala Phe Leu Val Arg Xaa Arg
               20                  25                  30
<210> 25
   <211> 31
   <212> PRT
   <213> Artificial Sequence
<220>
   <223> Description of Artificial Sequence: Synthetic polypeptide
<220>
   <221> MOD_RES
   <222> (2)..(2)
   <223> Ala, Val, or Gly
<220>
   <221> MOD_RES
   <222> (16)..(16)
   <223> Gly, Lys, or Glu
<220>
   <221> MOD_RES
   <222> (17)..(17)
   <223> Gln or Lys
<220>
   <221> MOD_RES
   <222> (24)..(24)
   <223> Ala or Glu
<220>
   <221> MOD_RES
   <222> (27)..(27)
   <223> Val or Lys
<220>
   <221> MOD_RES
   <222> (28)..(28)
   <223> Lys, Asn, or Arg
<220>
   <221> MOD_RES
   <222> (30)..(30)
   <223> Arg or Gly
<220>
   <221> MOD_RES
   <222> (31)..(31)
   <223> Gly, His, Pro, or not present
<220>
   <221> MOD_RES
   <222> (31)..(31)
   <223> AMIDATION (C-term may or may not be amidated)
<400> 25
   His Xaa Glu Gly Thr Phe Thr Ser Asp Val Ser Ser Tyr Leu Glu Xaa
   1               5                   10                  15
   Xaa Ala Ala Lys Glu Phe Ile Xaa Trp Leu Xaa Xaa Gly Xaa Xaa
               20                  25                  30
<210> 26
   <211> 31
   <212> PRT
   <213> Artificial Sequence
<220>
   <223> Description of Artificial Sequence: Synthetic polypeptide
<220>
   <221> MOD_RES
   <222> (31)..(31)
   <223> AMIDATION (C-term may or may not be amidated)
<400> 26
   His Ala Glu Gly Thr Phe Thr Ser Asp Val Ser Ser Tyr Leu Glu Gly
   1               5                   10                  15
   Gln Ala Ala Lys Glu Phe Ile Ala Trp Leu Val Arg Gly Arg Gly
               20                  25                  30
<210> 27
   <211> 31
   <212> PRT
   <213> Artificial Sequence
<220>
   <223> Description of Artificial Sequence: Synthetic polypeptide
<220>
   <221> MOD_RES
   <222> (31)..(31)
   <223> AMIDATION (C-term may or may not be amidated)
<400> 27
   His Ala Glu Gly Thr Phe Thr Ser Asp Val Ser Ser Tyr Leu Glu Gly
   1               5                   10                  15
   Gln Ala Ala Lys Glu Phe Ile Glu Trp Leu Val Lys Gly Arg Gly
               20                  25                  30
<210> 28
   <211> 31
   <212> PRT
   <213> Artificial Sequence
<220>
   <223> Description of Artificial Sequence: Synthetic polypeptide
<220>
   <221> MOD_RES
   <222> (31)..(31)
   <223> AMIDATION(C-term may or may not be amidated)
<400> 28
   His Ala Glu Gly Thr Phe Thr Ser Asp Val Ser Ser Tyr Leu Glu Lys
   1 5 10 15
   Gln Ala Ala Lys Glu Phe Ile Ala Trp Leu Val Lys Gly Arg Gly
   20 25 30
<210> 29
   <211> 31
   <212> PRT
   <213> Artificial Sequence
<220>
   <223> Description of Artificial Sequence: Synthetic polypeptide
<220>
   <221> MOD_RES
   <222> (31)..(31)
   <223> AMIDATION (C-term may or may not be amidated)
<400> 29
   His Gly Glu Gly Thr Phe Thr Ser Asp Val Ser Ser Tyr Leu Glu Glu
   1 5 10 15
   Gln Ala Ala Lys Glu Phe Ile Ala Trp Leu Val Lys Gly Gly Gly
   20 25 30
<210> 30
   <211> 31
   <212> PRT
   <213> Artificial Sequence
<220>
   <223> Description of Artificial Sequence: Synthetic polypeptide
<220>
   <221> MOD_RES
   <222> (31)..(31)
   <223> AMIDATION (C-term may or may not be amidated)
<400> 30
   His Val Glu Gly Thr Phe Thr Ser Asp Val Ser Ser Tyr Leu Glu Glu
   1 5 10 15
   Gln Ala Ala Lys Glu Phe Ile Ala Trp Leu Val Lys Gly Gly Gly
   20 25 30
<210> 31
   <211> 31
   <212> PRT
   <213> Artificial Sequence
<220>
   <223> Description of Artificial Sequence: Synthetic polypeptide
<220>
   <221> MOD_RES
   <222> (31)..(31)
   <223> AMIDATION (C-term may or may not be amidated)
<400> 31
   His Gly Glu Gly Thr Phe Thr Ser Asp Val Ser Ser Tyr Leu Glu Glu
   1               5                   10                  15
   Gln Ala Ala Lys Glu Phe Ile Ala Trp Leu Lys Asn Gly Gly Gly
               20                  25                  30
<210> 32
   <211> 31
   <212> PRT
   <213> Artificial Sequence
<220>
   <223> Description of Artificial Sequence: Synthetic polypeptide
<220>
   <221> MOD_RES
   <222> (31)..(31)
   <223> AMIDATION (C-term may or may not be amidated)
<400> 32
   His Val Glu Gly Thr Phe Thr Ser Asp Val Ser Ser Tyr Leu Glu Glu
   1               5                   10                  15
   Gln Ala Ala Lys Glu Phe Ile Ala Trp Leu Lys Asn Gly Gly Gly
               20                  25                  30
<210> 33
   <211> 31
   <212> PRT
   <213> Artificial Sequence
<220>
   <223> Description of Artificial Sequence: Synthetic polypeptide
<220>
   <221> MOD_RES
   <222> (31)..(31)
   <223> AMIDATION (C-term may or may not be amidated)
<400> 33
   His Gly Glu Gly Thr Phe Thr Ser Asp Val Ser Ser Tyr Leu Glu Glu
   1               5                   10                  15
   Gln Ala Ala Lys Glu Phe Ile Ala Trp Leu Val Lys Gly Gly Pro
               20                  25                  30
<210> 34
   <211> 31
   <212> PRT
   <213> Artificial Sequence
<220>
   <223> Description of Artificial Sequence: Synthetic polypeptide
<220>
   <221> MOD_RES
   <222> (31)..(31)
   <223> AMIDATION (C-term may or may not be amidated)
<400> 34
   His Val Glu Gly Thr Phe Thr Ser Asp Val Ser Ser Tyr Leu Glu Glu
   1               5                   10                  15
   Gln Ala Ala Lys Glu Phe Ile Ala Trp Leu Val Lys Gly Gly Pro
               20                  25                  30
<210> 35
   <211> 31
   <212> PRT
   <213> Artificial Sequence
<220>
   <223> Description of Artificial Sequence: Synthetic polypeptide
<220>
   <221> MOD_RES
   <222> (31)..(31)
   <223> AMIDATION (C-term may or may not be amidated)
<400> 35
   His Gly Glu Gly Thr Phe Thr Ser Asp Val Ser Ser Tyr Leu Glu Glu
   1               5                   10                  15
   Gln Ala Ala Lys Glu Phe Ile Ala Trp Leu Lys Asn Gly Gly Pro
               20                  25                  30
<210> 36
   <211> 31
   <212> PRT
   <213> Artificial Sequence
<220>
   <223> Description of Artificial Sequence: Synthetic polypeptide
<220>
   <221> MOD_RES
   <222> (31)..(31)
   <223> AMIDATION (C-term may or may not be amidated)
<400> 36
   His Val Glu Gly Thr Phe Thr Ser Asp Val Ser Ser Tyr Leu Glu Glu
   1 5 10 15
   Gln Ala Ala Lys Glu Phe Ile Ala Trp Leu Lys Asn Gly Gly Pro
   20 25 30
<210> 37
   <211> 30
   <212> PRT
   <213> Artificial Sequence
<220>
   <223> Description of Artificial Sequence: Synthetic polypeptide
<220>
   <221> MOD_RES
   <222> (30)..(30)
   <223> AMIDATION (C-term may or may not be amidated)
<400> 37
   His Gly Glu Gly Thr Phe Thr Ser Asp Val Ser Ser Tyr Leu Glu Glu
   1 5 10 15
   Gln Ala Ala Lys Glu Phe Ile Ala Trp Leu Val Lys Gly Gly
   20 25 30
<210> 38
   <211> 30
   <212> PRT
   <213> Artificial Sequence
<220>
   <223> Description of Artificial Sequence: Synthetic polypeptide
<220>
   <221> MOD_RES
   <222> (30)..(30)
   <223> AMIDATION (C-term may or may not be amidated)
<400> 38
   His Val Glu Gly Thr Phe Thr Ser Asp Val Ser Ser Tyr Leu Glu Glu
   1 5 10 15
   Gln Ala Ala Lys Glu Phe Ile Ala Trp Leu Val Lys Gly Gly
   20 25 30
<210> 39
   <211> 30
   <212> PRT
   <213> Artificial Sequence
<220>
   <223> Description of Artificial Sequence: Synthetic polypeptide
<220>
   <221> MOD_RES
   <222> (30)..(30)
   <223> AMIDATION (C-term may or may not be amidated)
<400> 39
   His Val Glu Gly Thr Phe Thr Ser Asp Val Ser Ser Tyr Leu Glu Glu
   1               5                   10                  15
   Gln Ala Ala Lys Glu Phe Ile Ala Trp Leu Val Asn Gly Gly
               20                  25                  30
<210> 40
   <211> 30
   <212> PRT
   <213> Artificial Sequence
<220>
   <223> Description of Artificial Sequence: Synthetic polypeptide
<220>
   <221> MOD_RES
   <222> (30)..(30)
   <223> AMIDATION (C-term may or may not be amidated)
<400> 40
   His Gly Glu Gly Thr Phe Thr Ser Asp Val Ser Ser Tyr Leu Glu Glu
   1               5                   10                  15
   Gln Ala Ala Lys Glu Phe Ile Ala Trp Leu Val Asn Gly Gly
               20                  25                  30
<210> 41
   <211> 230
   <212> PRT
   <213> Artificial Sequence
<220>
   <223> Description of Artificial Sequence: Synthetic polypeptide
<220>
   <221> MOD_RES
   <222> (16)..(16)
   <223> Pro or Glu
<220>
   <221> MOD_RES
   <222> (17)..(17)
   <223> Phe, Val, or Ala
<220>
   <221> MOD_RES
   <222> (18)..(18)
   <223> Leu, Glu, or Ala
<220>
   <221> MOD_RES
   <222> (80)..(80)
   <223> Asn or Ala
<220>
   <221> MOD_RES
   <222> (230)..(230)
   <223> May or may not be present
<400> 41
   Ala Glu Ser Lys Tyr Gly Pro Pro Cys Pro Pro Cys Pro Ala Pro Xaa
   1               5                   10                  15
   Xaa Xaa Gly Gly Pro Ser Val Phe Leu Phe Pro Pro Lys Pro Lys Asp
               20                  25                  30
   Thr Leu Met Ile Ser Arg Thr Pro Glu Val Thr Cys Val Val Val Asp
           35                  40                  45
   Val Ser Gln Glu Asp Pro Glu Val Gln Phe Asn Trp Tyr Val Asp Gly
       50                  55                  60
   Val Glu Val His Asn Ala Lys Thr Lys Pro Arg Glu Glu Gln Phe Xaa
   65                  70                  75                  80
   Ser Thr Tyr Arg Val Val Ser Val Leu Thr Val Leu His Gln Asp Trp
                   85                  90                  95
   Leu Asn Gly Lys Glu Tyr Lys Cys Lys Val Ser Asn Lys Gly Leu Pro
               100                 105                 110
   Ser Ser Ile Glu Lys Thr Ile Ser Lys Ala Lys Gly Gln Pro Arg Glu
           115                 120                 125
   Pro Gln Val Tyr Thr Leu Pro Pro Ser Gln Glu Glu Met Thr Lys Asn
       130                 135                 140
   Gln Val Ser Leu Thr Cys Leu Val Lys Gly Phe Tyr Pro Ser Asp Ile
   145                 150                 155                 160
   Ala Val Glu Trp Glu Ser Asn Gly Gln Pro Glu Asn Asn Tyr Lys Thr
                   165                 170                 175
   Thr Pro Pro Val Leu Asp Ser Asp Gly Ser Phe Phe Leu Tyr Ser Arg
               180                 185                 190
   Leu Thr Val Asp Lys Ser Arg Trp Gln Glu Gly Asn Val Phe Ser Cys
           195                 200                 205
   Ser Val Met His Glu Ala Leu His Asn His Tyr Thr Gln Lys Ser Leu
   210 215 220
   Ser Leu Ser Leu Gly Lys
   225 230
<210> 42
   <211> 30
   <212> PRT
   <213> Artificial Sequence
<220>
   <223> Description of Artificial Sequence: Synthetic polypeptide
<220>
   <221> misc_feature
   <222> (1)..(30)
   <223> This sequence may encompass 1-6 repeating "GGGGS" subunits
<400> 42
   Gly Gly Gly Gly Ser Gly Gly Gly Gly Ser Gly Gly Gly Gly Ser Gly
   1 5 10 15
   Gly Gly Gly Ser Gly Gly Gly Gly Ser Gly Gly Gly Gly Ser
   20 25 30
<210> 43
   <211> 275
   <212> PRT
   <213> Artificial Sequence
<220>
   <223> Description of Artificial Sequence: Synthetic polypeptide
<400> 43
   His Gly Glu Gly Thr Phe Thr Ser Asp Val Ser Ser Tyr Leu Glu Glu
   1 5 10 15
   Gln Ala Ala Lys Glu Phe Ile Ala Trp Leu Val Lys Gly Gly Gly Gly
   20 25 30
   Gly Gly Gly Ser Gly Gly Gly Gly Ser Gly Gly Gly Gly Ser Ala Glu
   35 40 45
   Ser Lys Tyr Gly Pro Pro Cys Pro Pro Cys Pro Ala Pro Glu Ala Ala
   50 55 60
   Gly Gly Pro Ser Val Phe Leu Phe Pro Pro Lys Pro Lys Asp Thr Leu
   65 70 75 80
   Met Ile Ser Arg Thr Pro Glu Val Thr Cys Val Val Val Asp Val Ser
   85 90 95
   Gln Glu Asp Pro Glu Val Gln Phe Asn Trp Tyr Val Asp Gly Val Glu
   100 105 110
   Val His Asn Ala Lys Thr Lys Pro Arg Glu Glu Gln Phe Asn Ser Thr
   115 120 125
   Tyr Arg Val Val Ser Val Leu Thr Val Leu His Gln Asp Trp Leu Asn
   130 135 140
   Gly Lys Glu Tyr Lys Cys Lys Val Ser Asn Lys Gly Leu Pro Ser Ser
   145 150 155 160
   Ile Glu Lys Thr Ile Ser Lys Ala Lys Gly Gln Pro Arg Glu Pro Gln
   165 170 175
   Val Tyr Thr Leu Pro Pro Ser Gln Glu Glu Met Thr Lys Asn Gln Val
   180 185 190
   Ser Leu Thr Cys Leu Val Lys Gly Phe Tyr Pro Ser Asp Ile Ala Val
   195 200 205
   Glu Trp Glu Ser Asn Gly Gln Pro Glu Asn Asn Tyr Lys Thr Thr Pro
   210 215 220
   Pro Val Leu Asp Ser Asp Gly Ser Phe Phe Leu Tyr Ser Arg Leu Thr
   225 230 235 240
   Val Asp Lys Ser Arg Trp Gln Glu Gly Asn Val Phe Ser Cys Ser Val
   245 250 255
   Met His Glu Ala Leu His Asn His Tyr Thr Gln Lys Ser Leu Ser Leu
   260 265 270
   Ser Leu Gly
   275
<210> 44
   <211> 40
   <212> PRT
   <213> Artificial Sequence
<220>
   <223> Description of Artificial Sequence: Synthetic polypeptide
<220>
   <221> MOD_RES
   <222> (2)..(2)
   <223> D-Ala, Gly, Val, Leu, Ile, Ser, or Thr
<220>
   <221> MOD_RES
   <222> (16)..(16)
   <223> Gly, Glu, Asp, or Lys
<220>
   <221> MOD_RES
   <222> (27)..(27)
   <223> Val or Ile
<220>
   <221> MOD_RES
   <222> (40)..(40)
   <223> C-term OH or NH2
<400> 44
   His Xaa Glu Gly Thr Phe Thr Ser Asp Val Ser Ser Tyr Leu Glu Xaa
   1               5                   10                  15
   Gln Ala Ala Lys Glu Phe Ile Ala Trp Leu Xaa Lys Gly Gly Pro Ser
               20                  25                  30
   Ser Gly Ala Pro Pro Pro Cys Cys
           35                  40
<210> 45
   <211> 40
   <212> PRT
   <213> Artificial Sequence
<220>
   <223> Description of Artificial Sequence: Synthetic polypeptide
<220>
   <221> MOD_RES
   <222> (40)..(40)
   <223> AMIDATION (C-term may or may not be amidated)
<400> 45
   His Val Glu Gly Thr Phe Thr Ser Asp Val Ser Ser Tyr Leu Glu Glu
   1               5                   10                  15
   Gln Ala Ala Lys Glu Phe Ile Ala Trp Leu Ile Lys Gly Gly Pro Ser
               20                  25                  30
   Ser Gly Ala Pro Pro Pro Cys Cys
           35                  40

## Description

### Field

The description is directed to the field of medicine, more particularly, to sleep.

### Background

Obstructive sleep apnea is caused by a blockage of the airway, which usually occurs when the soft tissue in the throat collapses and closes during sleep. The blockage can occur in a portion of the pharyngeal lumen and may include obstructions formed by the collapse of the tongue against the posterior wall of the pharynx, the collapse of the lateral pharyngeal walls, and the combined collapse of the tongue with impingement of the soft palate, particularly the posterior portion of the soft palate including the uvula. During each apnea event, the brain briefly arouses the sufferer in order to initiate the resumption of breathing. This type of sleep, however, is extremely fragmented and of poor quality.

According to the National Institutes of Health, obstructive sleep apnea affects more than twelve million Americans. Obstructive sleep apnea may result in high blood pressure, cardiovascular disease, weight gain, impotency, headaches, and memory problems if it is not treated. Despite the seriousness of obstructive sleep apnea, a general lack of awareness among the public and healthcare professionals results in the vast majority of obstructive sleep apnea sufferers remaining undiagnosed and untreated.

Many devices have been developed to treat obstructive sleep apnea; however, the devices can be uncomfortable for the patients. There exists a need in the art to identify therapeutic agents that can be used to enhance the quality of sleep and treat sleep apnea without causing patient discomfort. The present disclosure is directed to these, as well as other, important ends.

### Summary

Provided herein are compounds and compositions for use in the treatment of obstructive sleep apnea in patients in need thereof by administering to the patients therapeutically effective amounts of GLP-1 receptor agonist compounds to treat obstructive sleep apnea. The treatment of obstructive sleep apnea includes treatment for reducing the severity of obstructive sleep apnea.

In some instances, obstructive sleep apnea may be treated by administering BYETTA® (exenatide; Amylin Pharmaceuticals, Inc., San Diego, CA, and Eli Lilly and Co., Indianapolis, IN) to the patient. BYETTA® is a pharmaceutical composition that generally comprises a GLP-1 receptor agonist compound (e.g., exenatide), a preservative (e.g., metacresol), a tonicity-adjusting agent (e.g., mannitol), and a buffer (e.g., an acetate buffer).

In some instances, obstructive sleep apnea may be treated by administering BYDUREON™ or exenatide once weekly (EQW) (Amylin Pharmaceuticals, Inc., Eli Lilly and Company, Alkermes, Inc.) to the patient. Exenatide once weekly is a pharmaceutical composition that comprises biodegradable microspheres (e.g., poly(lactide-co-glycolide) microspheres) and exenatide. Exenatide once weekly is described, for example, in WO 2007/024700.

For the invention described herein, the patient may be a mammal, such as a human. Conditions such as obstructive sleep apnea are usually more common in diabetic and/or obese patients, such that the patient being treatedis more likely to be diabetic, obese, or diabetic and obese.

### Detailed Description

The description relates to GLP-1 receptor agonist compounds as defined in the claims for use to treat obstructive sleep apnea in patients in need thereof. The GLP-1 receptor agonist compounds are administered to patients in therapeutically effective amounts. The patient may be a mammal, such as a human. The description relates to pharmaceutical compositions comprising GLP-1 receptor agonist compounds for use to treat obstructive sleep apnea in patients in need thereof. The pharmaceutical compositions comprise therapeutically effective amounts of the GLP-1 receptor agonist compounds. The compositions may be immediate release (e.g., administered qid, bid, tid) or extended release (e.g., administered QW or once a month). The patient may be a mammal, such as a human.

In one embodiment, the description relates to GLP-1 receptor agonist compounds to treat obstructive sleep apnea by administering therapeutically effective amounts to patients in need thereof. A patient with obstructive sleep apnea stops breathing numerous times, for varying amounts of time, during sleep. Methods for treating obstructive sleep apnea include reducing the number of times a patient stops breathing during the night and/or reducing the length of time a patient stops breathing. "Reducing" includes eliminating the symptoms of obstructive sleep apnea.

The present invention provides a GLP-1 receptor agonist compound or a pharmaceutical composition comprising the GLP-1 receptor agonist compound for use in the treatment of obstructive sleep apnea in a patient in need thereof, said treatment comprising peripherally administering to the patient a therapeutically effective amount of the compound or pharmaceutical composition to treat the obstructive sleep apnea, wherein the GLP-1 receptor agonist compound is a GLP-1(7-37) analog ("GLP1(7-37)" disclosed as SEQ ID NO: 22); exendin-4 (SEQ ID NO:1); exendin-3 (SEQ ID NO:2); Leu¹⁴-exendin-4 (SEQ ID NO:3); Leu¹⁴,Phe²⁵-exendin-4 (SEQ ID NO:4); Leu¹⁴,Ala¹⁹,Phe²⁵-exendin-4 (SEQ ID NO:5); exendin-4(1-30) (SEQ ID NO:6); Leu¹⁴-exendin-4(1-30) (SEQ ID NO:7); Leu¹⁴,Phe²⁵-exendin-4(1-30) (SEQ ID NO:8); Leu¹⁴,Ala¹⁹,Phe²¹-exendin-4(1-30) (SEQ ID NO:9); exendin-4(1-28) (SEQ ID NO:10); Leu¹⁴-exendin-4(1-28) (SEQ ID NO:11); Leu¹⁴,Phe²⁵-exendin-4(1-28) (SEQ ID NO:12); Leu¹⁴Ala¹⁹,Phe²⁵-exendin-4 (1-28) (SEQ ID NO:13); Leu¹⁴,Lys^{17,20},Ala¹⁹,Glu²¹,Phe²⁵,Gln²⁸-exendin-4 (SEQ ID NO:14); Leu¹⁴,Lys^{17,20},Ala¹⁹,Glu²¹,Gln²⁸-exendin-4 (SEQ ID NO:15); octylGly¹⁴,Gln²⁸-exendin-4 (SEQ ID NO:16); Leu¹⁴,Gln²⁸,octylGly³⁴-exendin-4 (SEQ ID NO:17); Phe⁴,Leu¹⁴,Gln²⁸,Lys³³,Glu³⁴, Ile^{35,36},Ser³⁷-exendin-4(1-37) (SEQ ID NO:18); Phe⁴,Leu¹⁴,Lys^{17,20},Ala¹⁹,Glu²⁸,Gln²⁸-exendin-4 (SEQ ID NO:19); Val¹¹,Ile¹³,Leu¹⁴,Ala¹⁶,Lys²¹,Phe²⁵-exendin-4 (SEQ ID NO:20); exendin-4-Lys⁴⁰ (SEQ ID NO:21); lixisenatide; CJC-1134; [N^{ε}-(17-carboxyheptadecanoic acid)Lys²⁰]exendin-4-NH₂; [N^{ε}-(17-carboxyhepta-decanoyl)Lys³²]exendin-4-NH₂; [desamino-His¹,N^{ε}-(17-carboxyheptadecanoyl)Lys²⁰]exendin-4-NH₂; [Arg^{12,27},NLe¹⁴,N^{ε}-(17-carboxy-heptadecanoyl)Lys³²]exendin-4-NH2; [N^{ε}-(19-carboxy-nonadecanoylamino)Lys²⁰]-exendin-4-NH₂; [N^{ε}-(15-carboxypentadecanoylamino)Lys²⁰]-exendin-4-NH₂; [N^{ε}-(13-carboxytridecanoylamino)Lys²⁰]exendin-4-NH₂; [N^{ε}-(11-carboxy-undecanoyl-amino)Lys²⁰]exendin-4-NH₂; exendin-4-Lys⁴⁰(ε-MPA)-NH₂; exendin-4-Lys⁴⁰(ε-AEEA-AEEA-MPA)-NH₂; exendin-4-Lys⁴⁰(ε-AEEA-MPA)-NH₂; exendin-4-Lys⁴⁰(ε-MPA)-albumin; exendin-4-Lys⁴⁰(ε-AEEA-AEEA-MPA)-albumin; exendin-4-Lys⁴⁰(ε-AEEA-MPA)-albumin; or SEQ ID NO:43.

Obstructive sleep apnea may be classified by the Apnea-Hypopnea Index (AH1) which measures the total number of episodes of apnea and hypopnea occurring during sleep divided by the hours of sleep time. The AHI classifies sleep apnea as normal (i.e., less than 5 pauses in breathing lasting more than 10 seconds): mild (i.e., 5-15 pauses in breathing lasting more than 10 seconds); moderate (i.e., 15-30 pauses in breathing lasting more than 10 second); or severe (i.e., more than 30 pauses in breathing lasting more than 10 seconds). The treatment of sleep apnea described herein includes reducing the severity of obstructive sleep apnea based on the AHI. In one embodiment, the invention described herein reduces the patient's AHI from severe to moderate; or from severe to mild; or from severe to normal. In one embodiment, the invention described herein reduces the patient's AHI from moderate to mild, or from moderate to normal. In one embodiment, the methods described herein reduce the patient's AHI from mild to normal.

A "GLP-1 receptor agonist compound" refers to compounds having GLP-1 receptor activity. Such compounds are exendins, exendin analogs, and GLP-1(7-37) analogs.

The term "exendin" includes naturally occurring (or synthetic versions of naturally occurring) exendin peptides that are found in the salivary secretions of the Gila monster. Exendins of particular interest include exendin-3 and exendin-4. The exendins and exendin analogs for use in the methods described herein may optionally be amidated, and may also be in an acid form, pharmaceutically acceptable salt form, or any other physiologically active form of the molecule.

Exendin-4 (HGEGTFTSDLSKQMEEEAVRLFIEWLKNGGPSSGAPPPS-NH₂ (SEQ ID NO:1)) is a peptide found in the saliva of the Gila monster, *Heloderma suspectum*; and exendin-3 (HSDGTFTSDLSKQMEEEAVRLFIEWLKNGG PSSGAPPPS-NH₂ (SEQ ID NO:2)) is a peptide found in the saliva of the beaded lizard, *Heloderma horridum.* Exendins have some amino acid sequence similarity to some members of the glucagon-like peptide (GLP) family. For example, exendin-4 has about 53% sequence identity with glucagon-like peptide-1(GLP-1)(7-37) (HAEGTFTSDVSSYLEGQAAKEFIAWLVKGRG (SEQ ID NO:22)). However, exendin-4 is transcribed from a distinct gene, not the Gila monster homolog of the mammalian proglucagon gene from which GLP-1 is expressed. Additionally, exendin-4 is not an analog of GLP-1 (7-37) because the structure of synthetic exendin-4 peptide was not created by sequential modification of the structure of GLP-1. Nielsen et al, Current Opinion in Investigational Drugs, 4(4):401-405 (2003).

Synthetic exendin-4, also known as exenatide, is commercially available as BYETTA® (Amylin Pharmaceuticals, Inc. and Eli Lilly and Company). BYETTA® contains exenatide, a preservative (e.g., metacresol), a tonicity-adjusting agent (e.g., mannitol), and a buffer (e.g., an acetate buffer). A once weekly formulation of exenatide is currently in development and is described in WO 2005/102293. This once weekly formulation comprises exenatide and biodegradable polymeric (e.g., poly(lactide-co-glycolide)) microspheres, and is referred to herein as EQW or BYDUREON™ (Amylin Pharmaceuticals, Inc., Eli Lilly and Company, Alkermes, Inc.).

"Exendin analog" refers to peptides or other compounds which elicit a biological activity of an exendin reference peptide, preferably having a potency equal to or better than the exendin reference peptide (e.g., exendin-4), or within five orders of magnitude (plus or minus) of potency compared to the exendin reference peptide, when evaluated by art-known measures such as receptor binding and/or competition studies as described, e.g., by Hargrove et al, Regulatory Peptides, 141:113-119 (2007). Preferably, the exendin analogs will bind in such assays with an affinity of less than 1 µM, and more preferably with an affinity of less than 3 nM, or less than 1 nM. The term "exendin analog" may also be referred to as "exendin agonist".

Exendin analogs also include the peptides described herein which have been chemically derivatized or altered, for example, peptides with non-natural amino acid residues (e.g., taurine, β-amino acid residues, γ-amino acid residues, and D-amino acid residues), C-terminal functional group modifications, such as amides, esters, and C-terminal ketone modifications and N-terminal functional group modifications, such as acylated amines, Schiff bases, or cyclization, as found, for example, in the amino acid pyroglutamic acid. Exendin analogs may also contain other chemical moieties, such as peptide mimetics.

In one instance the term "exendin analog" refers to a peptide having at least 75% sequence identity to exendin-4. In other instances, the term "exendin analog" refers to peptides having at least 80% sequence identity to exendin-4; at least 85% sequence identity to exendin-4; at least 90% sequence identity to exendin-4; or at least 95% sequence identity to exendin-4.

Exemplary exendins and exendin analogs exendin-4 (SEQ ID NO:1); exendin-3 (SEQ ID NO:2); Leu¹⁴-exendin-4 (SEQ ID NO:3); Leu¹⁴,Phe²⁵-exendin-4 (SEQ ID NO:4); Leu¹⁴,Ala¹⁹,Phe²⁵-exendin-4 (SEQ ID NO:5); exendin-4(1-30) (SEQ ID NO:6); Leu¹⁴-exendin-4(1-30) (SEQ ID NO:7); Leu¹⁴,Phe²⁵-exendin-4(1-30) (SEQ ID NO:8); Leu¹⁴,Ala¹⁹,Phe²⁵-exendin-4(1-30) (SEQ ID NO:9); exendin4(1-28) (SEQ ID NO:10); Leu¹⁴-exendin-4(1-28) (SEQ ID NO:11); Leu¹⁴,Phe²⁵-exendin-4(1-28) (SEQ ID NO:12); Leu¹⁴,Ala¹⁹,Phe²⁵-exendin-4 (1-28)(SEQ ID NO:13); Leu¹⁴Lys^{17,20},Ala¹⁹,GlU²¹,Phe²⁵,Gln²⁸-exendin-4(SEQ ID NO:14); Leu¹⁴,Lys^{17,20},Ala¹⁹,Glu²¹,Gln²⁸-exendin-4 (SEQ ID NO:15); octylGly¹⁴,Gln²⁸-exendin-4 (SEQ ID NO:¹⁶); Leu¹⁴,Gln²⁸,octylGly³⁴-exendin-4 (SEQ ID NO:17); Phe⁴,Leu¹⁴,Gln²⁸,Lys³³,Glu³⁴, Ile^{35,36},Ser³⁷-exendin-4(1-37) (SEQ ID NO:18); Phe⁴,Leu¹⁴,Lys^{17,20},Ala¹⁹, Glu²¹,Gln²⁸-exendin-4 (SEQ ID NO:19); Val¹¹,Il¹³,Leu¹⁴,Ala¹⁶,Lys²¹,Phe²⁵-exendin-4 (SEQ ID NO:20); exendin-4-Lys⁴⁰ (SEQ ID NO:21); lixisenatide (Sanofi-Aventis/Zealand Pharma); CJC-1134 (ConjuChem, Inc.); [N^{ε}-(17-carboxyheptadecanoic acid)Lys²⁰]exendin-4-NH₂; [N^{ε}-(17-carboxyheptadecanoyl)Lys³²-]exendin-4-NH₂; [desamino-His¹,N^{ε}-(17-carboxyheptadecanoyl)Lys²⁰]exendin-4-NH₂; [Arg^{12,27},NLe¹⁴,N^{ε}-(17-carboxy-heptadecanoyl)Lys³²]exendin-4-NH₂; [N^{ε}-(19-carboxy-nonadecanoylamino)Lys²⁰]-exendin4-NH₂; [N^{ε}-(15-carboxypentadecanoylamino)Lys²⁰]-exendin-4-NH₂; [N^{ε}-(13-carboxytridecanoylamino)Lys²⁰exendin-4-NH₂; [N^{ε}-(11-carboxyundecanoyl-arnino)Lys²⁰]exendin-4-NH₂; excndin-4-Lys⁴⁰(ε-MPA)-NH₂; exendin-4-Lys⁴⁰(ε-AEEA-AEEA-MPA)-NH₂; exendin-4-Lys⁴⁰(ε-AEEA-MPA)-NH₂; exendin-4-Lys⁴⁰(ε-MPA)-albumin; exendin-4-Lys⁴⁰(ε-AEEA-AEEA-MPA)-albumin; exendin-4-Lys⁴⁰(ε-AEEA-MPA)-albumin; and the like. AEEA refers to [2-(2-amino)ethoxy)]ethoxy acetic acid. EDA refers to ethylenediamine. MPA refers to maleimidopropionic acid. The exendins and exendin analogs may optionally be amidated.

Other exendins and exendin analogs useful in the methods described herein include those described in WO 98/05351; WO 99/07404; WO 99/25727; WO 99/25728; WO 99/40788; WO 00/41546; WO 00/41548; WO 00/73331; WO 01/51078; WO 03/099314; US Patent No. 6,956,026; US Patent No. 6,506,724; US Patent No. 6,703,359; US Patent No. 6,858,576; US Patent No. 6,872,700; US Patent No. 6,902,744; US Patent No. 7,157,555; US Patent No. 7,223,725; US Patent No. 7,220,721; US Publication No. 2003/0036504; and US Publication No. 2006/0094652.

"GLP-1(7-37) analogs" refers to peptides or other compounds which elicit a biological activity similar to that of GLP-1(7-37), when evaluated by art-known measures such as receptor binding assays or in vivo blood glucose assays as described, e.g., by Hargrove et al, Regulatory Peptides, 141:113-119 (2007). In one instance, the term "GLP-1(7-37) analog" refers to a peptide that has an amino acid sequence with 1, 2, 3, 4, 5, 6, 7 or 8 amino acid substitutions, insertions, deletions, or a combination of two or more thereof, when compared to the amino acid sequence of GLP-1(7-37). In one instance, the GLP-1(7-37) analog is GLP-1(7-36)-NH₂. GLP-1(7-37) analogs include the amidated forms, the acid form, the pharmaceutically acceptable salt form, and any other physiologically active form of the molecule.

In one instance, the term "GLP-1(7-37)" refers to a peptide having at least 75% sequence identity to GLP-1(7-37). In other instances, the term "GLP-1(7-37) analog" refers to peptides having at least 80% sequence identity to GLP-1(7-37); at least 85% sequence identity to GLP-1(7-37); at least 90% sequence identity to GLP-1(7-37); or at least 95% sequence identity to GLP-1(7-37).

Exemplary GLP-1(7-37) and GLP-1(7-37) analogs include GLP-1(7-37) (SEQ ID NO:22); GLP-1(7-36) )-NH₂ (SEQ ID NO:23); liraglutide (VICTOZA® from Novo Nordisk); albiglutide (SYNCRIA® from GlaxoSmithKline); taspoglutide (Hoffman La-Roche); LY2189265 (Eli Lilly and Company); LY2428757 (Eli Lilly and Company); desamino-His⁷,Arg²⁶,Lys³⁴(N^{ε}-(γ-Glu(N-α-hexadecanoyl)))-GLP-1(7-37); desamino-His⁷,Arg²⁶,Lys³⁴(N^{ε}-octanoyl)-GLP-1(7-37); Arg^{26,34}Lys³⁸(N^{ε}-(ω-carboxypentadecanoyl))-GLP-1(7-38); Arg^{26,34},Lys³⁶(N^{ε}-(γ-Glu(N-α- hexadecanoyl)))-GLP-1(7-36); Aib^{8,35}Arg^{26,34},Phe³¹-GLP-1(7-36)) (SEQ ID NO:24); HXaa₈EGTFTSDVSSYLEXaa₂₂Xaa₂₃AAKEFIXaa₃₀WLXaa₃₃Xaa₃₄G Xaa₃₆Xaa₃₇; wherein Xaa₈ is A, V, or G; Xaa₂₂ is G, K, or E; Xaa₂₃ is Q or K; Xaa₃₀ is A or E; Xaa₃₃ is V or K; Xaa₃₄ is K, N, or R; Xaa₃₆ is R or G; and Xaa₃₇ is G, H, P, or absent (SEQ NO:25); Arg³⁴-GLP-1(7-37) (SEQ ID NO:26); Glu³⁰-GLP-1(7-37) (SEQ ID NO:27); Lys²²-GLP-1(7-37) (SEQ ID NO:28); Glyg^{8,26}Glu²²-GLP-1(7-37) (SEQ ID NO:29); Val⁸,Glu²²,Gly³⁶-GLP-1(7-37) (SEQ ID NO:30); Gly^{8,36},Glu²²,Lys³³,Asn³⁴-GLP-1(7-37) (SEQ ID NO:31); Val⁸,Glu²²,Lys³³,Asn³⁴,Gly³⁶-GLP-1(7-37) (SEQ ID NO:32); Gly^{8,36},Glu²²,Pro³⁷-GLP-1(7-37) (SEQ ID NO:33); Val⁸,Glu²²,Gly³⁶Pro³⁷-GLP-1(7-37) (SEQ ID NO:34); Gly^{8,36},Glu²²,Lys³³, Asn³⁴,Pro³⁷-GLP-1(7-37) (SEQ IDNO:35); Val⁸,Glu²²,Lys³³,Asn³⁴,Gly³⁶,Pro³⁷-GLP-1(7-37) (SEQ ID NO:36); Gly^{8,36},Glu²²-GLP-1(7-36) (SEQ ID NO:37); Val⁸,Glu²²,Gly³⁶-GLP-1(7-36) (SEQ ID NO:38); Val⁸,Glu²²,Asn³⁴,Gly³⁶-GLP-1(7-36) (SEQ ID NO:39); Gly^{8,36},Glu²²,Asn³⁴-GLP-1 (7-36) (SEQ ID NO:40). Each of the GLP-1(7-37) and GLP-1(7-37) analogs may optionally be amidated.

In one instance, the GLP-1(7-37) or GLP-1(7-37) analogs are covalently linked (directly or by a linking group) to an Fc portion of an immunoglobulin (e.g., IgG, IgE, IgG, and the like). For example, any one of SEQ ID NOs:25-40 may be covalently linked to the Fc portion of an immunoglobulin comprising the sequence of: AESKYGPPCPPCPAPXaa₁₆ Xaa₁₇Xaa₁₈GGPSVFLFPPKPKDTLMISRTPEVTCVVVDVSQEDPEVQFNWYVDGVEVH NAKTKPREEQFXaa₈₀STYRVVSVLTVLHQDWLNGKEYKCKVSNKGLPSSIEKTISKAKG QPREPQVYTLPPSQEEMTKNQVSLTCLVKGFYPSDIAVEWESNGQPENNYKTTPPVLD SDGSFFLYSRLTVDKSRWQEGNVFSCSVMHEALHNHYTQKSLSLSLGXaa₂₃₀; wherein Xaa₁₆ is P or E; Xaa₁₇ is F, V or A; Xaa₁₈ is L, E or A; Xaa₈₀ is N or A; and Xaa₂₃₀ is K or absent (SEQ ID NO:41). The linking group may be any chemical moiety (e.g., amino acids and/or chemical groups). In one instance, the linking group is (-GGGGS-)ₓ (SEQ ID NO:42) where x is 1, 2, 3, 4, 5 or 6; preferably 2, 3 or 4; more preferably 3. In one instance, the GLP-1(7-37) analog covalently linked to the Fc portion of an immunoglobulin comprises the amino acid sequence: HGEGTFTSDVSSYLEEQAAKEFIAWLVKGGGGGGGSGGGGSGGGGSA ESKYGPPCPPCPAPEAAGGPSVFLFPPKPKDTLMISRTPEVTCVVVDVSQEDPEVQ FNWYVDGVEVHNAKTKPREEQFNSTYRVVSVLTVLHQDWLNGKEYKCKVSN KGLPSSIEKTISKAKGQPREPQVYTLPPSQEEMTKNQVSLTCLVKGFYPSDIAVEWESN GQPENNYKTTPPVLDSDGSFFLYSRLTVDKSRWQEGNVFSCSVMHEALHNHYTQ KSLSLSLG (SEQ ID NO:43).

In another instance, the GLP-1(7-37) or GLP-1(7-37) analog may be covalently linked (directly or through a linking group) to one or two polyethylene glycol molecules. For example, a GLP-1(7-37) analog may comprise the amino acid sequence: HXaa₈EGTFTSDVS SYLEXaa₂₂AAKEFIAWLXaa₃₃KGGPSSGAPPPC₄₅C₄₆-Z, wherein Xaa₈ is: D-Ala, G, V, L, I, S or T; Xaa₂₂ is G, E, D or K; Xaa₃₃ is: V or I; and Z is OH or NH₂, (SEQ ID NO:44), and, optionally, wherein (i) one polyethylene glycol moiety is covalently attached to C₄₅, (ii) one polyethylene glycol moiety is covalently attached to C₄₆, or (iii) one polyethylene glycol moiety is attached to C₄₅ and one polyethylene glycol moiety is attached to C₄₆. In one instance, the GLP-1(7-37) analog is HVEGTFTSDVSSYLEEQAAKEFIAWLIKGGPSSGAPPPC₄₅C₄₆-N-H₂ (SEQ ID NO:45) and, optionally, wherein (i) one polyethylene glycol moiety is covalently attached to C₄₅, (ii) one polyethylene glycol moiety is covalently attached to C₄₆, or (iii) one polyethylene glycol moiety is attached to C₄₅ and one polyethylene glycol moiety is attached to C₄₆.

GLP-1 receptor agonist compounds may be prepared by processes well known in the art, e.g., peptide purification as described in Eng et al, J. Biol. Chem., 265:20259-62 (1990); standard solid-phase peptide synthesis techniques as described in Raufman et al, J. Biol. Chem., 267:21432-37 (1992); recombinant DNA techniques as described in Sambrook et al, Molecular Cloning: A Laboratory Manual, 2d Ed., Cold Spring Harbor (1989); and the like.

The disclosure also provides pharmaceutical compositions comprising the GLP-1 receptor agonist compounds described herein and a pharmaceutically acceptable carrier. The GLP-1 receptor agonist compounds can be present in the pharmaceutical composition in a therapeutically effective amount and can be present in an amount to provide a minimum blood plasma level of the GLP-1 receptor agonist compound necessary for therapeutic efficacy. Such pharmaceutical compositions are known in the art and described, e.g., in US Patent No. 7,521,423; US Patent No. 7,456,254; WO 2000/037098; WO 2005/021022; WO 2005/102293; WO 2006/068910; WO 2006/125763; WO 2009/068910; and US Publication No 2004/0106547.

Pharmaceutical compositions containing the GLP-1 receptor agonist compounds described herein are provided for peripheral administration, such as parenteral (e.g., subcutaneous, intravenous, intramuscular), a continuous infusion (e.g., intravenous drip, intravenous bolus, intravenous infusion), topical, nasal, or oral administration. Suitable pharmaceutically acceptable carriers and their formulation are described in standard formulation treatises, such as Remington's Pharmaceutical Sciences by Martin; and Wang et al, Journal of Parenteral Science and Technology, Technical Report No. 10, Supp. 42:2S (1988).

The GLP-1 receptor agonist compounds described herein can be provided in parenteral compositions for injection or infusion. They can, for example, be suspended in water; an inert oil, such as a vegetable oil (e.g., sesame, peanut, olive oil, and the like); or other pharmaceutically acceptable carrier. In one embodiment, the compounds are suspended in an aqueous carrier, for example, in an isotonic buffer solution at a pH of about 3.0 to 8.0, or about 3.0 to 5.0. The compositions may be sterilized by conventional sterilization techniques or may be sterile filtered. The compositions may contain pharmaceutically acceptable auxiliary substances as required to approximate physiological conditions, such as pH buffering agents. Useful buffers include for example, acetic acid buffers. A form of repository or "depot" slow release preparation may be used so that therapeutically effective amounts of the preparation are delivered into the bloodstream over many hours or days following subcutaneous injection, transdermal injection or other delivery method. The desired isotonicity may be accomplished using sodium chloride or other pharmaceutically acceptable agents such as dextrose, boric acid, sodium tartrate, propylene glycol, polyols (such as mannitol and sorbitol), or other inorganic or organic solutes. In one instance for intravenous infusion, the formulation may comprise (i) the GLP-1 receptor agonist compound, (2) sterile water, and, optionally (3) sodium chloride, dextrose, or a combination thereof.

Carriers or excipients can also be used to facilitate administration of the GLP-1 receptor agonist compounds. Examples of carriers and excipients include calcium carbonate, calcium phosphate, various sugars such as lactose, glucose, or sucrose, or types of starch, cellulose derivatives, gelatin, vegetable oils, polyethylene glycols and physiologically compatible solvents.

The GLP-1 receptor agonist compounds can also be formulated as pharmaceutically acceptable salts (e.g., acid addition salts) and/or complexes thereof. Pharmaceutically acceptable salts are non-toxic salts at the concentration at which they are administered. Pharmaceutically acceptable salts include acid addition salts such as those containing sulfate, hydrochloride, phosphate, sulfamate, acetate, citrate, lactate, tartrate, methanesulfonate, ethanesulfonate, benzenesulfonate, p-toluenesulfonate, cyclohexylsulfamate and quinate. Pharmaceutically acceptable salts can be obtained from acids such as hydrochloric acid, sulfuric acid, phosphoric acid, sulfamic acid, acetic acid, citric acid, lactic acid, tartaric acid, malonic acid, methanesulfonic acid, ethanesulfonic acid, benzenesulfonic acid, p-toluenesulfonic acid, cyclohexylsulfamic acid, and quinic acid. Such salts may be prepared by, for example, reacting the free acid or base forms of the product with one or more equivalents of the appropriate base or acid in a solvent or medium in which the salt is insoluble, or in a solvent such as water which is then removed in vacuo or by freeze-drying or by exchanging the ions of an existing salt for another ion on a suitable ion exchange resin.

Exemplary pharmaceutical formulations of GLP-1 receptor agonist compounds are described in US Patent No. 7,521,423, US Patent No. 7,456,254; US Publication No 2004/0106547, WO 2006/068910, and WO 2006/125763.

The therapeutically effective amount of the GLP-1 receptor agonist compounds described herein for use in the methods described herein will typically be from about 0.01 µg to about 5 mg; about 0.1 µg to about 2.5 mg; about 1 µg to about 1 mg; about 1 µg to about 50 µg; or about 1 µg to about 25 µg. Alternatively, the therapeutically effective amount of the GLP-1 receptor agonist compounds may be from about 0.001 µg to about 100 µg based on the weight of a 70 kg patient; or from about 0.01 µg to about 50 µg based on the weight of a 70 kg patient. These therapeutically effective doses may be administered once/day, twice/day, thrice/day, once/week, biweekly, or once/month, depending on the formulation. The exact dose to be administered is determined, for example, by the formulation, such as an immediate release formulation or an extended release formulation. For transdermal, nasal or oral dosage forms, the dosage may be increased from about 5-fold to about 10-fold.

In other instances of the invention described herein, the GLP-1 receptor agonist compound can be administered with (e.g., as separate compositions or in the same composition) an effective amount of an amylin, an amylin analog (e.g., davalintide), GIP, a GIP analog, PYY, a PYY analog, leptin, a leptin analog (e.g., metreleptin), or a combination of two or more thereof. Therapeutically effective amounts of these compounds are known in the art or can be determined by the skilled artisan based on the knowledge in the art and the teachings herein.

### Example

The example is for purposes of illustration only.

Thirty human patients with type 2 diabetes will take part in a six week, randomized, double-blind, placebo-controlled, parallel group study to determine the impact of exenatide on sleep and circadian function. The study will evaluate, e.g., minutes of NREM sleep; total amount of EEG slow-wave activity; habitual sleep duration and quality; and apnea-hypopnea index (AHI), a validated marker of the severity of obstructive sleep apnea.

Patients will be studied prior to randomization (baseline) and at the end of six weeks of treatment with twice daily subcutaneous injections of BYETTA® (exenatide; Amylin Pharmaceuticals, Inc. and Eli Lilly and Company) (5 µg for the first two week, and then 10 µg for the next four weeks) or placebo. An early assessment will be performed after 2 weeks of treatment under both conditions. The baseline study will include assessment of presence and severity of obstructive sleep apnea. The one week home studies will include recordings of habitual sleep duration and quality by wrist actigraphy and sleep logs, in addition to 3 days of meal logs.

Each second inpatient study will involve controlled caloric intake (matching the patient's daily requirements), continuous monitoring of interstitial glucose, two nights of polysomnography, and a 24 hour period of blood sampling at frequent intervals. A fasting blood sample will be drawn for the measurment of CRP, HbA1c, lipids, Apolipoproetin B, and adiponectin levels. The levels of plasma glucose, insulin, C-peptide, free fatty acids, leptin, ghrelin, PYY, melatonin, and catecholamines will be determined on the sample collected during 24 hour blood sampling. Additional measurements will include the 24 hour profile of heart rate variability and blood pressure, and frequent administration of scales to assess hunger, appetite, nausea, and daytime sleepiness. A biopsy of abdominal fat from the peri-umbilical region will be obtained. Tissue samples will be digested collagenase to isolate adipocytes. Insulin sensitivity will be determined by incubating the cells with increasing concentrations of insulin (0-10 nM). Cells will be stimulated for 10 minutes and analyzed by phospho-specific immunoblotting or for 45 minutes pluse 14C-glucose to measure lipogenesis. Alternatively, samples will be cultured in vitro as tissue chunks for analysis of oscillation of circadian gene expression over a 2 day period. Cells will be harvested periodically and gene expression determined by quantitative real time PCR.

The early assessment at 2 weeks after starting treatment will include a one week home study, discussed above, and a one day inpatient study with PSG to assess early effects of exenatide on sleep, CGMS, assessments of hunger, appetite, nausea, daytime sleepiness, 24 hour profile of heart rate variability and blood pressure, fasting blood samples for CRP, lipids, ApoB, and adiponectin.

The primary study endpoints are (i) minutes of non-REM sleep; (ii) total amount of EEG slow-wave activity; (iii) habitual sleep duration and quality; and (iv) AHI, a validated marker of the severity of obstructive sleep apnea. The second study enpoints include (i) glucose control as estimated by mean daytime, nighttime and 24 hour levels and mean peak post-prandial levels of interstitial glucose from 3 days of continuous blood glucose monitoring (CGMS) as well as HA1c; (ii) insulin sensitivity, as estimated by the fasting HOMA index, and insulin signaling directly measured in primary adipocytes using phosphospecific immunoblotting and metabolic assays; (iii) levels of hormones involved in the neuroendocrine control of appetite, including leptin, ghrelin and PYY; (iv) subjective hunger and appetite; (v) phase relationship between melatonin onset (marker of central circadian rhythmicity) and nocturnal peak of leptin profile (marker of peripheral rhythm); and (vi) rhythm of circadian gene expression in primary adipocytes.

## Claims

1. A GLP-1 receptor agonist compound or a pharmaceutical composition comprising a GLP-1 receptor agonist compound for use in the treatment of obstructive sleep apnea in a patient in need thereof, said treatment comprising peripherally administering to the patient a therapeutically effective amount of the compound or pharmaceutical composition to treat the obstructive sleep apnea, wherein the GLP-1 receptor agonist compound is a GLP-1(7-37) analog ("GLP1(7-37)" disclosed as SEQ ID NO: 22); exendin-4 (SEQ ID NO:1); exendin-3 (SEQ ID NO:2); Leu¹⁴-exendin-4 (SEQ ID NO:3); Leu¹⁴,Phe²⁵-exendin-4 (SEQ ID NO:4); Leu¹⁴,Ala¹⁹,Phe²⁵-exendin-4 (SEQ ID NO:5); exendin-4(1-30) (SEQ ID NO:6); Leu¹⁴-exendin-4(1-30) (SEQ ID NO:7); Leu¹⁴,Phe²⁵-exendin-4(1-30) (SEQ ID NO:8); Leu¹⁴,Ala¹⁹,Phe²⁵-exendin-4(1-30) (SEQ ID NO:9); exendin-4(1-28) (SEQ ID NO:10); Leu¹⁴-exendin-4(1-28) (SEQ ID NO:11); Leu¹⁴,Phe²⁵-exendin-4(1-28) (SEQ ID NO:12); Leu¹⁴,Ala¹⁹,Phe²⁵-exendin-4 (1-28) (SEQ ID NO:13); Leu¹⁴,Lys^{17,20},Ala¹⁹,Glu²¹,Phe²⁵,Gln²⁸-exendin-4 (SEQ ID NO:14); Leu¹⁴,Lys^{17,20},Ala¹⁹,Glu²¹,Gln²⁸-exendin-4 (SEQ ID NO:15); octylGly¹⁴,Gln²⁸-exendin-4 (SEQ ID NO:16); Leu¹⁴,Gln²⁸,octylGly³⁴-exendin-4 (SEQ ID NO:17); Phe⁴,Leu¹⁴,Gln²⁸,Lys³³,Glu³⁴, Ile^{35,36},Ser³⁷-exendin-4(1-37) (SEQ ID NO:18); Phe⁴,Leu¹⁴,Lys^{17,20},Ala¹⁹,Glu²⁸,Gln²⁸-exendin-4 (SEQ ID NO:19); Val¹¹,Ile¹³,Leu¹⁴,Ala¹⁶,Lys²¹,Phe²⁵-exendin-4 (SEQ ID NO:20); exendin-4-Lys⁴⁰ (SEQ ID NO:21); lixisenatide; CJC-1134; [N^{ε}-(17-carboxyheptadecanoic acid)Lys²⁰]exendin-4-NH₂; [N^{ε}-(17-carboxyheptadecanoyl)Lys³²]exendin-4-NH₂; [desamino-His¹,N^{ε}-(17-carboxyheptadecanoyl)Lys²⁰]exendin-4-NH₂; [Arg^{12,27},NLe¹⁴,N^{ε}-(17-carboxy-heptadecanoyl)Lys³²]exendin-4-NH₂; [N^{ε}-(19-carboxynonadecanoylamino)Lys²⁰]-exendin-4- NH₂; [N⁶-(15-carboxypentadecanoylamino)Lys²⁰]-exendin-4-NH₂; [N^{ε}-(13-carboxytridecanoylamino)Lys²⁰]exendin-4-NH₂; [N^{ε}-(11-carboxy-undecanoyl-amino)Lys²⁰]exendin-4-NH₂; exendin-4-Lys⁴⁰(ε-MPA)-NH₂; exendin-4-Lys⁴⁰(ε-AEEA-AEEA-MPA)-NH₂; exendin-4-Lys⁴⁰(ε-AEEA-MPA)-NH₂; exendin-4-Lys⁴⁰(ε-MPA)-albumin; exendin-4-Lys⁴⁰(ε-AEEA-AEEA-MPA)-albumin; exendin-4-Lys⁴⁰(ε-AEEA-MPA)-albumin; or SEQ ID NO:43.

2. The compound or pharmaceutical composition for use according to claim 1, wherein the GLP-1 receptor agonist compound is GLP-1(7-37) (SEQ ID NO:22); GLP-1(7-36) (SEQ ID NO:23); liraglutide; albiglutide; taspoglutide; LY2189265; LY2428757; desamino-His⁷,Arg²⁶,Lys³⁴(N^{ε}-(γ-Glu(N-α-hexadecanoyl)))-GLP-1(7-37); desamino-His⁷,Arg²⁶,Lys³⁴(N^{ε}-octanoyl)-GLP-1(7-37); Arg^{26,34},Lys³⁸(N^{ε}-(ω-carboxypentadecanoyl))-GLP-1(7-38); Arg^{26,34},Lys³⁶(N^{ε}-(γ-Glu(N-α- hexadecanoyl)))-GLP-1(7-36); Aib^{8,35},Arg^{26,34},Phe³¹-GLP-1(7-36)) (SEQ ID NO:24); HXaa₈EGTFTSDVSSYLEXaa₂₂Xaa₂₃AAKEFIXaa₃₀WLXaa₃₃Xaa₃₄G Xaa₃₆Xaa₃₇; wherein Xaa₈ is A, V, or G; Xaa₂₂ is G, K, or E; Xaa₂₃ is Q or K; Xaa₃₀ is A or E; Xaa₃₃ is V or K; Xaa₃₄ is K, N, or R; Xaa₃₆ is R or G; and Xaa₃₇ is G, H, P, or absent (SEQ ID NO:25); Arg³⁴-GLP-1(7-37) (SEQ ID NO:26); Glu³⁰-GLP-1(7-37) (SEQ ID NO:27); Lys²²-GLP-1(7-37) (SEQ ID NO:28); Gly^{8,36},Glu²²-GLP-1(7-37) (SEQ ID NO:29); Val⁸,Glu²²,Gly³⁶-GLP-1(7-37) (SEQ ID NO:30); Gly^{8,36},Glu²²,Lys³³,Asn³⁴-GLP-1(7-37) (SEQ ID NO:31); Val⁸,Glu²²,Lys³³,Asn³⁴,Gly³⁶-GLP-1(7-37) (SEQ ID NO:32); Gly^{8,36},Glu²²,Pro³⁷-GLP-1(7-37) (SEQ ID NO:33); Val⁸,Glu²²,Gly³⁶Pro³⁷-GLP-1(7-37) (SEQ ID NO:34); Gly^{8,36},Glu²²,Lys³³, Asn³⁴,Pro³⁷-GLP-1(7-37) (SEQ ID NO:35); Val⁸,Glu,²²,Lys³³,Asn³⁴,Gly³⁶,Pro³⁷-GLP-1(7-37) (SEQ ID NO:36); Gly^{8,36},Glu²²-GLP-1(7-36) (SEQ ID NO:37); Val⁸,Glu²²,Gly³⁶-GLP-1(7-36) (SEQ ID NO:38); Val⁸,Glu²²,Asn³⁴,Gly³⁶-GLP-1(7-36) (SEQ ID NO:39); or Gly^{8,36},Glu²²,Asn³⁴-GLP-1(7-36) (SEQ ID NO:40).

3. The compound or pharmaceutical composition for use according to claim 1, wherein the GLP-1 receptor agonist compound is any one of SEQ ID NOs:25-40 covalently linked to the Fc portion of an immunoglobulin comprising the sequence of: wherein Xaa₁₆ is P or E; Xaa₁₇ is F, V or A; Xaa₁₈ is L, E or A; Xaa₈₀ is N or A; and Xaa₂₃₀ is K or absent (SEQ ID NO:41).

4. The compound or pharmaceutical composition for use according to claim 1, wherein the GLP-1 receptor agonist compound is exendin-4 (SEQ ID NO:1).

## Patentansprüche

1. GLP-1-Rezeptor-Agonist-Verbindung oder eine eine GLP-1-Rezeptor-Agonist-Verbindung umfassende pharmazeutische Zusammensetzung zur Verwendung bei der Behandlung von obstruktiver Schlafapnoe bei einem diese benötigenden Patienten, wobei die Behandlung das periphere Verabreichen einer therapeutisch wirksamen Menge der Verbindung oder pharmazeutischen Zusammensetzung an den Patienten umfasst, um die obstruktive Schlafapnoe zu behandeln, wobei die GLP-1-Rezeptor-Agonist-Verbindung ist: ein GLP-1 (7-37)-Analogon ("GLP1 (7-37)", offenbart als SEQ ID NO: 22); Exendin-4 (SEQ ID NO: 1); Exendin-3 (SEQ ID NO: 2); Leu¹⁴-Exendin-4 (SEQ ID NO: 3); Leu¹⁴, Phe²⁵-Exendin-4 (SEQ ID NO: 4); Leu¹⁴, Ala¹⁹, Phe²⁵-Exendin-4 (SEQ ID NO: 5); Exendin-4(1-30) (SEQ ID NO: 6); Leu¹⁴-Exendin-4(1-30) (SEQ ID NO: 7); Leu¹⁴, Phe²⁵-Exendin-4( 1-30) (SEQ ID NO: 8); Leu¹⁴, Ala¹⁹,Phe²⁵-Exendin-4(1-30) (SEQ ID NO: 9); Exendin-4(1-28) (SEQ ID NO: 10); Leu¹⁴-Exendin-4(1-28) (SEQ ID NO: 11); Leu¹⁴, Phe²⁵-Exendin-4(1-28) (SEQ ID NO: 12); Leu¹⁴, Ala¹⁹ ,Phe²⁵-Exendin-4 (1-28) (SEQ ID NO: 13); Leu¹⁴, Lys^{17,20},Ala¹⁹,Glu²¹,Phe²⁵, Gln²⁸-Exendin-4 (SEQ ID NO: 14); Leu¹⁴, Lys^{17,20}, Ala¹⁹, Glu²¹, Gln²⁸-Exendin-4 (SEQ ID NO: 15); octylGly¹⁴, Gln²⁸-Exendin-4 (SEQ ID NO: 16); Leu¹⁴,Gln²⁸,octylGly³⁴-Exendin-4 (SEQ ID NO: 17); Phe⁴,Leu¹⁴,Gln²⁸,Lys³³,Glu³⁴,Ile^{35,36},Ser³⁷-Exendin-4(1-37) (SEQ ID NO: 18); Phe⁴,Leu¹⁴,Lys^{17,20},Ala¹⁹,Glu²¹,Gln²⁸-Exendin-4 (SEQ ID NO: 19); Val¹¹,Ile¹³,Leu¹⁴,Ala¹⁶,Lys²¹,Phe²⁵-Exendin-4 (SEQ ID NO: 20); Exendin-4- Lys⁴⁰ (SEQ ID NO: 21); Lixisenatid; CJC-1134; [N^{ε}-(17-Carboxyheptadecansäure)Lys²⁰]exend in-4-NH₂; [N^{ε}-(17-Carboxyhepta-decanoyl)Lys³²]exendin-4-NH₂; [Desamino-His¹,N^{ε}-(17-carboxyheptadecanoyl)Lys²⁰]exendin-4-NH₂; [Arg^{12,27},NLe¹⁴,N^{ε}-(17-carboxy-heptadecanoyl)Lys³²]exendin-4-NH₂; [N^{ε}-(19-carboxy-nonadecanoylamino)Lys²⁰]-exendin-4-NH₂; [N^{ε}-(15-carboxypentadecanoylamino)Lys²⁰]-exendin-4-NH₂; [N^{ε}-(13-carboxytridecanoylamino)Lys²⁰]exendin-4-NH₂; [N^{ε}-(11-carboxy-undecanoyl-amino)Lys²⁰]exendin-4-NH₂; Exendin-4-Lys⁴⁰(ε-MPA)-NH₂; Exendin-4-Lys⁴⁰(ε-AEEA-AEEA-MPA)-NH₂; Exendin-4-Lys⁴⁰(ε-AEEA-MPA)-NH₂; Exendin-4-Lys⁴⁰(e-MPA)-albumin; Exendin-4-Lys⁴⁰(ε-AEEA-AEEA-MPA)-albumin; Exendin-4-Lys⁴⁰(ε-AEEA-MPA)-albumin; oder SEQ ID NO: 43.

2. Verbindung oder pharmazeutische Zusammensetzung zur Verwendung nach Anspruch 1, wobei die GLP-1-Rezeptor-Agonist-Verbindung ist: GLP-1 (7-37) (SEQ ID NO: 22); GLP-1 (7-36) (SEQ ID NO: 23); Liraglutid; Albiglutid; Taspoglutid; LY2189265; LY2428757; Desamino-His⁷,Arg²⁶,Lys³⁴ (N^{ε}-(γ-Glu(N-α-hexadecanoyl)))-GLP-1(7-37); Desamino-His⁷,Arg²⁶,Lys³⁴ (N^{ε}-octanoyl)-GLP-1 (7-37); Arg^{26,34},Lys³⁸(N^{ε}-((ω-carboxypentadecanoyl))-GLP-1(7-38); Arg^{26,34},Lys³⁶(N^{ε}-(γ-Glu(N-α-hexadecanoyl)))-GLP-1(7-36); Aib^{8,35},Arg^{26,34},Phe³¹-GLP-1(7-36)) (SEQ ID NO: 24); HXaa₈EGTFTSDVSSYLEXaa₂₂Xaa₂₃AAKEFIXaa₃₀WLXaa₃₃Xaa₃₄G Xaa₃₆Xaa₃₇; wobei Xaa₈ A, V oder G ist; Xaa₂₂ G, K oder E ist; Xaa₂₃ Q oder K ist; Xaa₃₀ A oder E ist; Xaa₃₃ V oder K ist; Xaa₃₄ K, N oder R ist; Xaa₃₆ R oder G ist; und Xaa₃₇ G, H, P ist oder fehlt (SEQ ID NO: 25); Arg³⁴-GLP-1(7-37) (SEQ ID NO: 26); Glu³⁰-GLP-1(7-37) (SEQ ID NO: 27); Lys²²-GLP-1(7-37) (SEQ ID NO: 28); Gly^{8,36},Glu²²-GLP-1(7-37) (SEQ ID NO: 29); Val⁸,Glu²²,Gly³⁶-GLP-1(7-37) (SEQ ID NO: 30); Gly^{8,36},Glu²²,Lys³³,Asn³⁴-GLP-1(7-37) (SEQ ID NO: 31); Val⁸,Glu²²,Lys³³,Asn³⁴,Gly³⁶-GLP-1(7-37) (SEQ ID NO: 32); Gly^{8,36},Glu²²,Pro³⁷-GLP-1 (7-37) (SEQ ID NO: 33); Val⁸,Glu²²,Gly³⁶Pro³⁷-GLP-1(7-37) (SEQ ID NO: 34); Gly^{8,36},Glu²²,Lys³³,Asn³⁴,Pro³⁷-GLP-1(7-37) (SEQ ID NO: 35); Val⁸,Glu²²,Lys³³,Asn³⁴,Gly³⁶,Pro³⁷-GLP-1(7-37) (SEQ ID NO: 36); Gly^{8,36},Glu²²-GLP-1(7-36) (SEQ ID NO: 37); Val⁸,Glu²²,Gly³⁶-GLP-1(7-36) (SEQ ID NO: 38); Val⁸,Glu²²,Asn³⁴,Gly³⁶-GLP-1(7-36) (SEQ ID NO: 39); oder Gly^{8,36},Glu²²,Asn³⁴-GLP-1(7-36) (SEQ ID NO: 40).

3. Verbindung oder pharmazeutische Zusammensetzung zur Verwendung nach Anspruch 1, wobei die GLP-1-Rezeptor-Agonist-Verbindung eine der SEQ ID NO: 25-40 ist, die kovalent mit dem Fc-Abschnitt eines Immunglobins mit der Sequenz: verbunden ist;
wobei Xaa₁₆ P oder E ist; Xaa₁₇ F, V oder A ist; Xaa₁₈ L, E oder A ist; Xaa₈₀ N oder A ist; und Xaa₂₃₀ K ist oder fehlt (SEQ ID NO: 41).

4. Verbindung oder pharmazeutische Zusammensetzung zur Verwendung nach Anspruch 1, wobei die GLP-1-Rezeptor-Agonist-Verbindung Exendin-4 (SEQ ID NO: 1) ist.

## Revendications

1. Composé agoniste du récepteur du GLP-1 ou composition pharmaceutique comprenant un composé agoniste du récepteur du GLP-1 destiné(e) à être utilisé(e) dans le traitement de l'apnée obstructive du sommeil chez un patient en ayant besoin, ledit traitement comprenant une administration périphérique au patient d'une quantité thérapeutiquement efficace du composé ou de la composition pharmaceutique pour traiter l'apnée obstructive du sommeil, où le composé agoniste du récepteur du GLP-1 est un analogue du GLP-1(7-37) (« GLP1 (7-37) » décrit en tant que SEQ ID NO: 22) ; l'exendine-4 (SEQ ID NO: 1) ; l'exendine-3 (SEQ ID NO: 2) ; Leu¹⁴-exendine-4 (SEQ ID NO: 3) ; Leu¹⁴,Phe²⁵-exendine-4 (SEQ ID NO: 4) ; Leu¹⁴,Ala¹⁹,Phe²⁵-exendine-4 (SEQ ID NO: 5) ; l'exendine-4(1-30) (SEQ ID NO: 6) ; Leu¹⁴-exendine-4(1-30) (SEQ ID NO: 7) ; Leu¹⁴,Phe²⁵-exendine-4((1-30) (SEQ ID NO: 8) ; Leu¹⁴,Ala¹⁹,Phe²⁵-exendine-4(1-30) (SEQ ID NO: 9) ; l'exendine-4(1-28) (SEQ ID NO: 10) ; Leu¹⁴-exendine-4(1-28) (SEQ ID NO: 11) ; Leu¹⁴,Phe²⁵-exendine-4(1-28) (SEQ ID NO: 12) ; Leu¹⁴,Ala¹⁹,Phe²⁵-exendine-4(1-28) (SEQ ID NO: 13) ; Leu¹⁴,Lys^{17,21},Ala¹⁹,Glu²¹,Phe²⁵,Gln²⁸-exendine-4 (SEQ ID NO: 14) ; Leu¹⁴, Lys^{17,20},Ala¹⁹,Glu²¹,Gln²⁸-exendine-4 (SEQ ID NO: 15) ; octylGly¹⁴,Gln²⁸-exendine-4 (SEQ ID NO: 16) ; Leu¹⁴, Gln²⁸, octylGly³⁴-exendine-4 (SEQ ID NO: 17) ; Phe⁴, Leu¹⁴, Gln²⁸, Lys³³, Glu³⁴, Ile^{35,31}, Ser³⁷-exendine-4 (1-37) (SEQ ID NO: 18) ; Phe⁴,Leu¹⁴,Lys^{17,20},Ala¹⁹,Glu²¹,Gln²⁰-exendine-4 (SEQ ID NO: 19) ; Val¹¹, Ile¹³,Leu¹⁴,Ala¹⁶,Lys²¹,Phe²⁵-exendine-4 (SEQ ID NO: 20) ; l'exendine-4-Lys⁴⁰ (SEQ ID NO: 21) ; le lixisénatide ; CJC-1134 ; [N^{ε}-(acide 17-carboxyheptadécanoïque)Lys²⁰] exendine-4-NH₂ ; [N^{ε}-(17-carboxyheptadécanoyl)Lys³²]exendine-4-NH₂; [désamino-His¹,N^{ε}-(17-carboxyheptadécanoyl)Lys²⁰]exendine-4-NH₂ ; [Arg^{12,27},NLe¹⁴,N^{ε}-(17-carboxy-heptadécanoyl)Lys³²]exendine-4-NH₂ ; [N^{ε}-(19-carboxy-nonadécanoylamino)Lys²⁰]exendine-4-NH₂ ; [N^{ε}-(15-carboxypentadécanoylamino)Lys²⁰]exendine-4-NH₂ ; [N²-(13-carboxytridécanoylamino)Lys²⁰]exendine-4-NH₂ ; [N^{ε}-(11-carboxy-undécanoyl-amino)Lys²⁰]exendine-4-NH2 ; l'exendine-4-Lys⁴⁰(ε-MPA)-NH₂ ; l'exendine-4-Lys⁴⁰(ε-AEEA-AEEA-MPA)-NH₂ ; l'exendine-4-Lys⁴⁰(ε-AEEA-MPA)-NH₂ ; l'exendine-4-Lys⁴⁰(ε-MPA)-albumine ; l'exendine-4-Lys⁴⁰(ε-AEEA-AEEA-MPA)-albumine ; l'exendine-4-Lys⁴⁰(ε-AEEA-MPA)-albumine ; ou SEQ ID NO: 43.

2. Composé ou composition pharmaceutique destiné (e) à être utilisé (e) selon la revendication 1, où le composé agoniste du récepteur du GLP-1 est GLP-1(7-37) (SEQ ID NO: 22) ; GLP-1(7-36) (SEQ ID NO: 23) ; le liraglutide ; l'albiglutide ; le taspoglutide ; LY2189265 ; LY2428757 ; désamino-His⁷,Arg²⁶,Lys³⁴(N^{ε}-(γ-Glu(N-α-hexadécanoyl)))-GLP-1(7-37) ; désamino-His⁷,Arg²⁶,Lys³⁴(N^{ε}-octanoyl)-GLP-1(7-37) ; Arg^{26,34},Lys³⁸(N^{ε}-(ω-carboxypentadécanoyl))-GLP-1(7-38) ; Arg^{26,34},Lys³⁶(N^{ε}-(γ-Glu(N-α-hexadécanoyl)))-GLP-1(7-36) ; Aib^{8,35},Arg^{26,34},Phe³¹-GLP-1(7-36)) (SEQ ID NO: 24) ; HXaa₈EGTFTSDVSSYLEXaa₂₂Xaa₂₃AAKEFIXaa₃₀WLXaa₃₃Xaa₃₄GXaa₃₆Xa a₃₇ ; où Xaa₈ est A, V, ou G ; Xaa₂₂ est G, K, ou E ; Xaa₂₃ est Q ou K ; Xaa₃₀ est A ou E ; Xaa₃₃ est V ou K ; Xaa₃₄ est K, N, ou R ; Xaa₃₆ est R ou G ; et Xaa₃₇ est G, H, P, ou absent (SEQ ID NO: 25) ; Arg³⁴-GLP-1(7-37) (SEQ ID NO: 26) ; Glu³⁰-GLP-1(7-37) (SEQ ID NO: 27) ; Lys²²-GLP-1(7-37) (SEQ ID NO: 28) ; Gly^{8,36},Glu²²-GLP-1(7-37) (SEQ ID NO: 29) ; Val⁸, Glu²²,Gly³⁶-GLP-1(7-37) (SEQ ID NO: 30) ; Gly^{8,36},Glu²²,Lys³³,Asn³⁴-GLP-1(7-37) (SEQ ID NO: 31) ; Val⁸,Glu²²,Lys³³,Asn³⁴,Gly³⁶-GLP-1(7-37) (SEQ ID NO: 32) ; Gly^{8,36},Glu²²,Pro³⁷-GLP-1(7-37) (SEQ ID NO: 33) ; Val⁸, Glu²²,Gly³⁶Pro³⁷-GLP-1(7-37) (SEQ ID NO: 34) ; Gly^{8,36},Glu²²,Lys³³,Asn³⁴,Pro³⁷-GLP-1(7-37) (SEQ ID NO: 35) ; Val⁸,Glu²²,Lys³³,Asn³⁴,Gly³⁶,Pro³⁷-GLP-1(7-37) (SEQ ID NO: 36) ; Gly^{8,36},Glu²²-GLP-1(7-36) (SEQ ID NO: 37) ; Val⁸,Glu²²,Gly³⁶-GLP-1(7-36) (SEQ ID NO: 38) ; Val⁸,Glu²²,Asn³⁴,Gly³⁶-GLP-1(7-36) (SEQ ID NO: 39) ; ou Gly^{8,36},Glu²²,Asn³⁴-GLP-1(7-36) (SEQ ID NO: 40).

3. Composé ou composition pharmaceutique destiné (e) à être utilisé(e) selon la revendication 1, où le composé agoniste du récepteur du GLP-1 est l'une quelconque de SEQ ID NO: 25-40 liée de façon covalente à la partie Fc d'une immunoglobuline comprenant la séquence de :
AESKYGPPCPPCPAPXaa₁₆Xaa₁₇Xaa₁₈GGPSVFLFPPKPKDTLMISRTPEVTCV VVDVSQEDPEVQFNWYVDGVEVHNAKTKPREEQFXaa₈₀STYRVVSVLTVLHQDW LNGKEYKCKVSNKGLPSSIEKTISKAKGQPREPQVYTLPPSQEEMTKNQVSLTCL VKGFYPSDIAVEWESNGQPENNYKTTPPVLDSDGSFFLYSRLTVDKSRWQEGNVF SCSVMHEALHNHYTQKSLSLSLGXaa₂₃₀ ; où Xaa₁₆ est P ou E ; Xaa₁₇ est F, V ou A ; Xaa₁₈ est L, E ou A ; Xaa₈₀ est N ou A ; et Xaa₂₃₀ est K ou absent (SEQ ID NO: 41).

4. Composé ou composition pharmaceutique destiné (e) à être utilisé(e) selon la revendication 1, où le composé agoniste du récepteur du GLP-1 est l'exendine-4 (SEQ ID NO: 1).
